# EUROPEAN PATENT SPECIFICATION

(11) **EP 1 200 093 B1**
(45) Date of publication and mention of the grant of the patent: **24.09.2003**
(21) Application number: 00949718.1
(22) Date of filing: 28.07.2000
(51) Int. Cl.: A61K 31/505, A61P 1/00

(54) **USE OF 2-AMINO-4-(4-FLUORONAPHTH-1-YL)-6-ISOPROPYLPYRIMIDINE IN THE TREATMENT OF GI DISORDERS**
VERWENDUNG VON 2-AMINO-4-(4-FLUORONAPHTH-1-YL)-6-ISOPROPYLPYRIMIDINE ZUR BEHANDLUNG VON ERKRANKUNGEN DES MAGEN-DARM-TRAKTES
UTILISATION DE 2-AMINO-4-(4-FLUORONAPHTH-1-YL)-6-ISOPROPYLPYRIMIDINE POUR LE TRAITEMENT DES TROUBLES GASTRO-INTESTINAUX

(30) Priority: 30.07.1999 GB 9918058; 11.02.2000 GB 0003228
(43) Date of publication of application: 02.05.2002
(73) Proprietor: Pharmagene Laboratories Ltd, Royston, Herts SG8 5HD (GB)
(72) Inventor: BAXTER, Gordon, Smith, Pharmagene Lab. Ltd, Royston, Hertfordshire SG8 5HD (GB); BORMAN, Richard, Anthony, Pharmagene Lab. Ltd, Royston, Hertfordshire SG8 5HD (GB)
(74) Representative: Brasnett, Adrian Hugh
(86) International application number: GB0002907
(87) International publication number: WO01008668

(56) References cited:
- WO-A-97/44326
- US-A- 5 863 924
- US-A- 5 952 331
- US-A- 5 958 934
- MANGEL A W ET AL: "Review article: The safety and efficacy of alosetron, a 5-HT3 receptor antagonist, in female irritable bowel syndrome patients." ALIMENTARY PHARMACOLOGY & THERAPEUTICS, vol. 13, no. SUPPL. 2, May 1999 (1999-05), pages 77-82, XP000980361 ISSN: 0269-2813
- HUMPHREY P P A ET AL: "Review article: The therapeutic potential of 5-HT3 receptor antagonists in the treatment of irritable bowel syndrome." ALIMENTARY PHARMACOLOGY & THERAPEUTICS, vol. 13, no. SUPPL. 2, May 1999 (1999-05), pages 31-38, XP000980388 ISSN: 0269-2813
- KISHIBAYASHI NOBUYUKI ET AL: "5-HT-3 receptor antagonists: 3. Quinoline derivatives which may be effective in the therapy of irritable bowel syndrome." JOURNAL OF MEDICINAL CHEMISTRY, vol. 36, no. 22, 1993, pages 3286-3292, XP002158321 ISSN: 0022-2623
- BONHAUS DOUGLAS W ET AL: "RS-127445: A selective, high affinity, orally bioavailable 5-HT2B receptor antagonist." BRITISH JOURNAL OF PHARMACOLOGY, vol. 127, no. 5, July 1999 (1999-07), pages 1075-1082, XP000980373 ISSN: 0007-1188

## Description

The present invention relates to medicaments for the treatment of irritable bowel syndrome (IBS).

### Background to the invention.

Serotonin, also referred to as 5-hydroxytryptamine (5-HT), is a neurotransmitter with mixed and complex pharmacological characteristics. 5-HT acts via a number of discrete 5-HT receptors. Currently, fourteen subtypes of serotonin receptor are recognized and delineated into seven families, 5-HT₁ to 5-HT₇. Within the 5-HT₂ family, 5-HT_{2A}, 5-HT_{2B} and 5-HT_{2C} subtypes are known to exist. The nomenclature and classification of 5-HT receptors has been reviewed by Martin and Humphrey, Neuropharm., 33, 261-273 (1994) and Hoyer et al., Pharm. Rev., 46, 157-203 (1994).

5-HT_{2B} receptors, initially termed 5-HT2F or serotonin-like receptor, were first characterized in rat isolated stomach fundus (Clineschmidt et al., J. Pharmacol. Exp. Ther., 235, 696-708 (1985) and Cohen and Wittenauer, J. Cadiovasc. Pharmacol., 10, 176-181 (1987)).

WO97/44326 and US 5,863,924 describe aryl pyrimidine derivatives and their use as selective 5-HT_{2B} antagonists. Of the numerous compounds described, 2-amino-4-(4-fluoronaphth-1-yl)-6-isopropylpyrimidine is mentioned. Bonhaus et al, Br. J. Pharmacol. 1999, 127, 1075-1082, report that this compound (RS127445) is a selective, high affinity, 5-HT_{2B} receptor antagonist.
IBS is a common disorder, characterised by chronic and recurrent gastrointestinal symptoms such as altered bowel function, pain, flatulence, bloating and constipation and/or diarrhoea, but for which there is no apparent biochemical or structural cause. IBS is not associated with any general deterioration in health. While the cause of IBS is unknown, the condition is often associated with stress or anxiety.

Humphrey et al, Aliment.Pharmacol.Ther. 1999;(13):31-38 review the therapeutic potential of 5-HT₃ receptor antagonists in the treatment of IBS.

Current approaches to the treatment of IBS include the antagonism of 5-HT at 5-HT₃ receptors, and either mimicking or blocking the effects of 5-HT at 5-HT₄ receptors in the gastrointestinal tract. Development of 5-HT₄ and 5-HT₃ antagonists was based upon observations in animal studies, where both types of receptors are present on myenteric neurones, and mediate the enhancement of the release of excitatory neurotransmitters which cause contraction of gastrointestinal smooth muscle.

### Disclosure of the invention.

The present invention is based upon the finding that whereas current approaches to the treatment of IBS include the antagonism of 5-HT at 5-HT₃ receptors, and either mimicking or blocking the effects of 5-HT at 5-HT₄ receptors in the gastrointestinal tract, we have found that in the human GI tract S-HT_{2B} receptors are present on myenteric neurones, and smooth muscle and that these receptors mediate the enhancement of the effects of neuronal stimulation, causing a potentiation of the contraction of GI smooth muscle that results from stimulation of the myenteric neurones. This potentiation of the neuronally-mediated contractile response may be inhibited or eliminated by the application of antagonists at 5-HT_{2B} receptors. The localisation of functional 5-HT_{2B} receptors on human myenteric neurones has not previously been demonstrated in humans or indeed in any other species.

The invention further provides the use of 2-amino-4-(4-fluoronaphth-1-yl)-6-isopropylpyrimidine, or a salt or N-oxide thereof, in the manufacture of a medicament for the treatment of IBS (irritable bowel syndrome).

The compound may be in the form of a composition comprising said compound together with a pharmaceutically acceptable diluent or carrier.

### Detailed description of the invention.

### IBS.

The diagnosis of IBS is difficult due to the plethora of symptoms and the lack of reliable diagnostic tests. Diagnosis, is often made on the basis of careful questioning of the patient but in patients with diarrhoea, other conditions such as inflammatory bowel disease (IBD), infective gastroenteritis, and bowel carcinoma need to be excluded. In addition, as it is so common, IBS can often co-exist with other disorders, a fact that needs to be recognised by physicians. In 1989, internationally agreed diagnostic criteria for IBS were drawn up by a group of renowned experts. These are known as the Rome I criteria and are frequently used by clinicians and researchers world-wide (Thompson W.G., Dotevall G., Drossman D.A. et al., 1989, IBS: guidelines for the diagnosis. Gastroenterology 2: 92-95). They are essentially the same as those proposed by Manning et al (Manning A.P., Thompson W.G., Heaton K.W. & Morris A.F., 1978, Towards positive diagnosis of the irritable bowel. Br. Med. J. 2, 653-4). See also Camilleri and Prather, 1992, The irritable bowel syndrome: mechanisms and a practical approach to management. Ann. Intern. Med. 116, 1001-8).

In summary, the criteria are as follows:

### Clinical features

A. Continuous or recurrent side effects for 3 months or more, viz.:
   1. Abdominal pain, relieved with defecation, or associated with a change in frequency or consistency of stool, AND/OR
B. Irregular or varying side effects 25% or more of the time including 3 or more of the following:
   1. Altered stool frequency
   2. Altered stool consistency
   3. Altered stool passage (straining or urgency, feeling of incomplete evacuation)
   4. Passage of mucus
   5. Bloating or feeling of abdominal distension.

### Effective amount.

The effective amount of the compound or its salt to be administered will ultimately be at the discretion of the physician, taking into account the severity of the disease in a particular subject (e.g. a human patient or animal model) and the overall condition of the subject. Suitable dose ranges will typically be in the range of from 0.01 to 20 mg/kg/day, preferably from 0.1 to 10 mg/kg/day.

### 2-Amino-4-(4-fluoronaphth-1-yl)-6-isopropylpyrimidine.

This compound may be manufactured by reference to WO97/44326.

### A salt or N-oxide of 2-amino-4-(4-fluoronaphth-1-yl)-6-isopropylpyrimidine.

A salt of this compound may be an acid addition salt in which the base retains the biological effectiveness and properties of the compound and which is physiologically acceptable. Such salts include those formed with inorganic acids such as hydrochloric acid, hydrobromic acid, sulfuric acid, nitric acid, phosphoric acid and the like, and organic acids such as acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methanesulfonic acid, ethanesulfonic acid, p-toluenesulfonic acid, salicylic acid and the like.

"N-oxide" refers to the stable amine oxide formed at one of the pyrimidine nitrogen atoms.

Acid addition salts and N-oxides may be produced by means conventional in the art.

### Medicaments.

This term refers to any medicament containing the compound, salt or N-oxide thereof, intended to alleviate the severity of IBS in a subject, and includes medicaments intended to cure the disease, provide relief from the symptoms of the disease and to prevent or arrest the development of the disease in an individual at risk from developing the disease or an individual having symptoms indicating the development of the disease in that individual.

### Compositions and their administration.

Compositions may be formulated for any suitable route and means of administration. Pharmaceutically acceptable carriers or diluents include those used in formulations suitable for oral, rectal, nasal, topical (including buccal and sublingual), vaginal or parenteral (including subcutaneous, intramuscular, intravenous, intradermal, intrathecal and epidural) administration. The formulations may conveniently be presented in unit dosage form and may be prepared by any of the methods well known in the art of pharmacy. Such methods include the step of bringing into association the active ingredient with the carrier which constitutes one or more accessory ingredients. In general the formulations are prepared by uniformly and intimately bringing into association the active ingredient with liquid carriers or finely divided solid carriers or both, and then, if necessary, shaping the product.

For solid compositions, conventional non-toxic solid carriers include, for example, pharmaceutical grades of mannitol, lactose, cellulose, cellulose derivatives, starch, magnesium stearate, sodium saccharin, talcum, glucose, sucrose, magnesium carbonate, and the like may be used. The active compound as defined above may be formulated as suppositories using, for example, polyalkylene glycols, acetylated triglycerides and the like, as the carrier. Liquid pharmaceutically administrable compositions can, for example, be prepared by dissolving, dispersing, etc, an active compound as defined above and optional pharmaceutical adjuvants in a carrier, such as, for example, water, saline aqueous dextrose, glycerol, ethanol, and the like, to thereby form a solution or suspension. If desired, the pharmaceutical composition to be administered may also contain minor amounts of non-toxic auxiliary substances such as wetting or emulsifying agents, pH buffering agents and the like, for example, sodium acetate, sorbitan monolaurate, triethanolamine sodium acetate, sorbitan monolaurate, triethanolamine oleate, etc. Actual methods of preparing such dosage forms are known, or will be apparent, to those skilled in this art; for example, see Remington's Pharmaceutical Sciences, Mack Publishing Company, Easton, Pennsylvania, 15th Edition, 1975. The composition or formulation to be administered will, in any event, contain a quantity of the active compound(s) in an amount effective to alleviate the symptoms of the subject being treated.

Dosage forms or compositions containing active ingredient in the range of 0.25 to 95% with the balance made up from non-toxic carrier may be prepared.

For oral administration, a pharmaceutically acceptable non-toxic composition is formed by the incorporation of any of the normally employed excipients, such as, for example, pharmaceutical grades of mannitol, lactose, cellulose, cellulose derivatives, sodium crosscarmellose, starch, magnesium stearate, sodium saccharin, talcum, glucose, sucrose, magnesium, carbonate, and the like. Such compositions take the form of solutions, suspensions, tablets, pills, capsules, powders, sustained release formulations and the like. Such compositions may contain 1%-95% active ingredient, more preferably 2-50%, most preferably 5-8%.

Parenteral administration is generally characterized by injection, either subcutaneously, intramuscularly or intravenously. Injectables can be prepared in conventional forms, either as liquid solutions or suspensions, solid forms suitable for solution or suspension in liquid prior to injection, or as emulsions. Suitable excipients are, for example, water, saline, dextrose, glycerol, ethanol or the like. In addition, if desired, the pharmaceutical compositions to be administered may also contain minor amounts of non-toxic auxiliary substances such as wetting or emulsifying agents, pH buffering agents and the like, such as for example, sodium acetate, sorbitan monolaurate, triethanolamine oleate, triethanolamine sodium acetate, etc.

A more recently devised approach for parenteral administration employs the implantation of a slow-release or sustained-release system, such that a constant level of dosage is maintained. See, e.g., US Patent No. 3,710,795.

The percentage of active compound contained in such parental compositions is highly dependent on the specific nature thereof, as well as the activity of the compound and the needs of the subject. However, percentages of active ingredient of 0.1% to 10% in solution are employable, and will be higher if the composition is a solid which will be subsequently diluted to the above percentages. Preferably, the composition will comprise 0.2-2% of the active agent in solution.

The following example illustrates the invention.

### Materials and Methods

Strips of human colon longitudinal muscle were prepared as follows:

A section of human colon was cut open along its longitudinal axis. The section was pinned out flat and the mucosa carefully removed using sharp dissecting scissors. Once the mucosa was removed, the section was turned over to reveal the three taenia coli (taenia mesencolica, taenia omentalis and taenia libera) and the muscle bands that lie between them. Longitudinal muscle strips (2mm wide by 20mm long) were then cut from the tissue between the taenia coli and suspended between stainless steel hooks in organ chambers containing oxygenated (95% O₂/5% CO₂) Krebs solution at 37°C. The composition of the Krebs solution was as follows: NaCI (118.2mM), KCI (4.69mM), MgsO₄₋7H₂O (1.18mM), KH₂PO₄ (1.19mM), glucose (11.1mM), NaHCO₃ (25.0mM), CaCI₂₋6H₂O (2.5mM).

Tissues were placed under a tension equivalent to 10mN and left to equilibrate for a period of at least 60 minutes. Responses were recorded using isometric transducers coupled to an Apple Macintosh computer via a MacLab interface. After 60 minutes, the longitudinal muscle sections of the human colon were stimulated electrically (sub-maximal voltage and frequency with 60s between successive stimulations), using parallel platinum wire electrodes and a Multistim D330 pulse stimulator. Upon electrical stimulation, the strips of human colon longitudinal smooth muscle responded with a rapid contraction.

Once the response to electrical stimulation had stabilised (successive stimulated responses differed by no more than 10%), the strips of human colon longitudinal smooth muscle were exposed to increasing concentrations of 5-HT or 5-HT receptor agonist, or 5-HT in the presence of 5-HT receptor antagonist.

Potent enhancement of electrically-induced contractions of longitudinal muscle strips from human colon by low concentrations of 5-HT (about 10⁻⁹ to 1O-⁷M) was observed.

### Reference Example 1 - Effects of 5-HT receptor agonists.

Application of 5-HT, as well as various 5-HT receptor agonists, has been shown to produce a potent enhancement of the contractile response to electrical stimulation. The electrically-induced contractile response is inhibited by both tetrodotoxin and atropine, indicating that the effect is neuronal in nature, and is mediated via the release of acetylcholine.

The order of agonist potency at enhancing the contractile response in colon longitudinal muscle was determined according to the method outlined above. The results are shown in Table 1, and compared to the mean pEC₅₀ for agonists at the 5-HT receptor in colon (mean ± s.e.mean for at least 3 donors) and also at human S-HT_{2A, 2B}, _{2C} and S-HT₄ receptors whereby all data are for human 5-HT receptors. NA indicates that data are not available at the human receptor.

**Table 1**

| Table 1. Potencies of some selective receptor agonists at the 5-HT receptor in human colon longitudinal muscle. | | | | | |
|---|---|---|---|---|---|
| **Agonist** | **Colon** | **5-HT**_{**2A**} | **5-HT**_{**2B**} | **5-HT**_{**2C**} | **5-HT**_{**4**} |
| Alpha-Me-5-HT | 8.5±0.1 | NA | 8.4 | NA | 6.2 |
| 5-HT | 8.2±0.1 | 7.4 | 8.1 | 8.5 | 6.7 |
| 2-Me-5-HT | 7.0±0.4 | NA | 6.7 | NA | 6.0 |
| 5-MeOT | 6.8±0.2 | NA | 7.6 | NA | 6.2 |
| Cisapride | 5.8±0.3 | NA | NA | NA | 5.5 |

The order of agonist potency implicates a receptor of the 5-HT₂ family.

### Reference Example 2 - Effects of 5-HT receptor antagonists.

The effects of various selective 5-HT receptor antagonists against 5-HT induced activity was also tested, according to the methods set out above. The affinities of selective receptor antagonists at the 5-HT receptor in human colon longitudinal muscle was determined (for at least 3 donors) and compared to the mean pK_{B} or pA₂ for antagonism at human S-HT_{2A}, _{2B}, _{2C} and 5-HT₄ receptors. The results are shown in Table 2. Responses to 5-HT was measured in human isolated tissues whose 5-HT receptor characteristics have been described previously (*) or from binding data at human recombinant receptors (#). NA indicates data are not available at the human receptor, NSA indicates non-surmountable antagonism. The profile generated corresponds to a receptor of the 5-HT_{2B} receptor class.

**Table 2**

| Table 2. Affinities of some selective receptor antagonists at the 5-HT receptor in human colon longitudinal muscle and comparisons. | | | | | |
|---|---|---|---|---|---|
| **Antagonist** | **Colon** | **5-HT**_{**2A**} | **5-HT**_{**2B**} | **5-HT**_{**2C**} | **5-HT**_{**4**} |
| SB 206553 | 8.5±0.1 | 6.0# | 9.0* | 7.9-9.0# | NA |
| Rauwolscine | 7.9±0.2 | 6.6# | 7.0-7.8# | 5.8# | NA |
| Yohimbine | 7.5±0.2 | 5.3# | 8.0* | <5.0# | NA |
| Methiothepin | 7.5±0.3 | NA | 8.1# | NA | <6.0* |
| Cisapride | 7.1±0.1 | 8.0# | 7.2# | 6.3# | NA |
| SB 204741 | 6.8±0.2 | 5.2# | 6.7# | 5.8# | <5.0 |
| SB 242084 | 6.3±0.2 | 6.8# | 7.0# | <6.0# | <5.0# |
| Ketanserin | 6.1±0.2 | 8.6* | <6.0* | 6.8* | 4.7* |
| Methysergide | NSA at -9M | 8.4# | 9.6* | 8.9* | <5.0* |

### Example - Effect of RS127445 on colon longitudinal muscle.

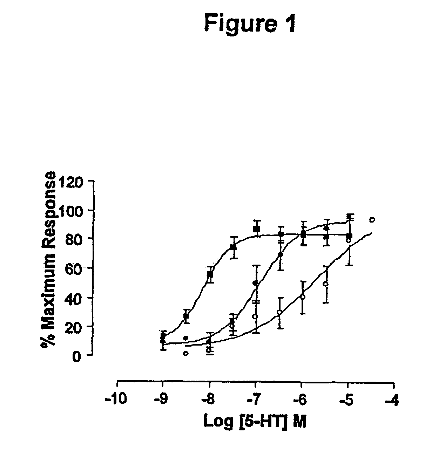

Concentrations between 0.3nM and 100nM of RS127445 were found to antagonise the effects of 5-HT in electrically-stimulated longitudinal muscle strips of human colon (experiments performed as described above) with a pK_{B} of 9.5 (see Table 3). The antagonism produced by RS127445 is shown in Figure 1, which shows the response to 5-HT in the absence (control, ■) and presence of RS127445 at a concentration of 1nM (●) and 100nM (o). All data are expressed as a percentage of the maximum response to 5-HT, and are given as meant s.e. mean for n>4 donors. Schild analysis of this antagonism yielded a plot with slope of 0.93±0.17, in accordance with competitive antagonism. Thus RS127445 is a potent (pK_{B} = 9.45±0.41) and selective antagonist of the effects of 5-HT at the 5-HT_{2B} receptor in human colon, showing that this compound may be used in the treatment of IBS and other GI disorders.

**Table 3**

| **Antagonist** | **Colon** | **5-HT**_{**2A**} | **5-HT**_{**2B**} | **5-HT**_{**2C**} | **5-HT**_{**4**} |
|---|---|---|---|---|---|
| RS 127445 | 9.45±0.41 | 6.3±0.2 | 9.5±0.1 | 6.4±0.1 | NA |

### Summary of Examples.

In summary, the functional ligand profile at the receptor that mediates 5-HT-induced potentiation of the neuronally-mediated contractile response in human colon corresponds to that at the 5-HT_{2B} receptor sub-type. This indicates therefore that a selective antagonist at this receptor is able to counteract the hypermotility effects of 5-HT in the human intestine, and thereby represents an effective treatment for IBS. RS127445 (2-amino-4-(4-fluoronaphth-1-yl)-6-isopropylpyrimidine) is both more potent than other antagonists on colon longitundinal muscle, and more selective for the 2B receptor subtype compared to the 2A and 2C receptor subtypes than other antagonists. Thus the use of RS127445, for on manufacture of a medicament for the treatment of IBS is indicated by the present novel findings.

## Claims

1. The use of 2-amino-4-(4-fluoronaphth-1-yl)-6-isopropylpyrimidine, or a salt or N-oxide thereof, in the manufacture of a medicament for the treatment of IBS (irritable bowel syndrome)

2. Use according to claim 1 wherein said salt is the hydrochloride or maleate salt.

3. Use according to claim 1 or 2 wherein said compound is in the form of a composition comprising said compound together with a pharmaceutically acceptable diluent or carrier.

## Patentansprüche

1. Verwendung von 2-Amino-4-(4-fluornaphth-1-yl)-6-isopropylpyrimidin oder eines Salzes oder N-Oxids davon, bei der Herstellung eines Medikaments zur Behandlung von Reizdarmsyndrom ("irritable bowel syndrome", IBS).

2. Verwendung nach Anspruch 1, worin das Salz das Hydrochlorid- oder Maleatsalz ist.

3. Verwendung nach Anspruch 1 oder 2, worin die Verbindung in Form einer Zusammensetzung vorliegt, die die Verbindung gemeinsam mit einem pharmazeutisch annehmbaren Verdünner oder Träger umfasst.

## Revendications

1. Utilisation de 2-amino-4-(4-fluoronapht-1-yl)-6-isopropylpyrimidine, ou un sel ou N-oxyde de celle-ci, dans la fabrication d'un médicament pour le traitement de SII (syndrome des intestins irritables).

2. Utilisation selon la revendication 1, où ledit sel est le sel de chlorhydrate ou de maléate.

3. Utilisation selon la revendication 1 ou 2, où ledit composé est sous la forme d'une composition comprenant ledit composé avec un diluant ou support pharmaceutiquement acceptable.
